# FASCICULE DE BREVET EUROPEEN

(11) **EP 0 452 239 B1**
(45) Date de publication et mention de la délivrance du brevet: **26.07.1995**
(21) Numéro de dépôt: 91460021.8
(22) Date de dépôt: 10.04.1991
(51) Int. Cl.: H04M 1/26, A61F 4/00

(54) **Système de commande multifonctions comportant des moyens électro-acoustiques de sélection de fonctions activables par un opérateur et des moyens de validation de la sélection**
Nehrfachsfunktionssteuerungssystem mit elektroakustischen Mitteln zur Auswahl von durch einen Benutzer aktivierbaren Funktionen und Mittel zur Gültigkeitserklärung dieser Auswahl
Multifunction control system with electroacoustic means for selecting available functions by an operator and means for validating the selection

(30) Priorité: 10.04.1990 FR 9004786
(43) Date de publication de la demande: 16.10.1991
(73) Titulaire: FRANCE TELECOM, 75015 Paris (FR)
(72) Inventeur: Perignon, Marc, F-22700 Louannec (FR); Daouben, Jean, F-22560 Trebeurden (FR)
(74) Mandataire: Corlau, Vincent

(56) Documents cités:
- AU-B- 557 720
- FR-A- 2 640 405
- GB-A- 2 006 495
- US-A- 3 725 602
- US-A- 3 836 959
- US-A- 4 275 266
- US-A- 4 562 432
- BELL LABORATORIES RECORD, vol. 57, no. 9, octobre 1973, pages 272-277, Bell Inc., Murray Hill, NJ, US; S.C. KITSOPOULOS et al.: "Experimental telephone lets disabled "dial" by voice"

## Description

Le domaine de l'invention est celui des dispositifs de commande d'appareils à partir de moyens d'activation autres que des contacts tactiles ou manuels du type boutons-poussoirs, interrupteurs, etc...

Plus précisément, la présente invention concerne un dispositif de commande d'appareils par le son, et notamment le souffle, pouvant en particulier être utilisé par des handicapés.

La mise en service d'appareils constitue en effet un problème majeur pour les personnes handicapées moteur.

La commande manuelle d'appareils est également inadaptée dans certaines situations rencontrées par des personnes parfaitement valides, par exemple lorsqu'il est nécessaire d'effectuer plusieurs tâches simultanément en milieu professionnel, ou plus simplement pour des raisons de confort (commande à distance ou sans effort musculaire).

Pour résoudre ce problème, on a développé des dispositifs à commande vocale. Ainsi, il existe déjà des dispositifs utilisant la reconnaissance de la parole pour mettre en (ou hors) service des appareils, ou pour réaliser une fonction particulière. Cependant, ces dispositifs ne fonctionnent pas de façon satisfaisante en milieu bruyant. Deux types de problèmes peuvent en effet se poser : la non-détection de commandes couvertes par le bruit et la détection de fausses commandes, par assimilation d'un bruit intempestif à une commande.

Il en va de même des systèmes utilisant la reconnaissance de sons (tel un sifflement, comme décrit par exemple dans le document de brevet anglais GB-A-2.006.495), qui ne peuvent convenir à l'activation et/ou la désactivation d'appareils dans un environnement bruyant.

On connaît également des systèmes de commande à capteurs de souffle, tels que décrits dans le document de brevet français FR-A-2 614 161 déposé le 15 avril 1987, et concernant un "dispositif téléphonique". Ce document décrit un dispositif détectant le souffle de l'utilisateur à l'aide de capteurs à commande mécanique ou magnétique. On sait en effet que le souffle présente un spectre de fréquences très large, ce qui en facilite la détection, même en présence d'un bruit ambiant. Ces capteurs de souffle présentent cependant des inconvénients tenant notamment à leur usure mécanique, à la spécificité de leur application, etc...

Un autre dispositif de commande par le souffle est décrit dans le document de brevet américain US-A-4.562.432 délivré le 31 décembre 1985 et qui concerne un dispositif permettant d'activer ou de désactiver des appareils correspondant chacun à un canal du dispositif. Le numéro de chaque canal apparaît sur un afficheur pendant un certain temps suivant un cycle continu déroulant. La détection d'un signal par un capteur de souffle active (ou désactive) l'appareil branché sur le canal dont le numéro est affiché. Ce dispositif s'avère relativement figé. L'utilisateur n'a pas la possibilité d'agir sur le rythme du défilement des numéros des canaux, ce rythme étant fonction d'une horloge interne au dispositif.

Tous ces dispositifs connus permettent, d'une façon ou d'une autre, la sélection d'une fonction par l'émission d'un son, ou d'un souffle. De ce fait, il présente une grande sensibilité aux bruits extérieurs. Par exemple, une porte qui claque, ou une personne qui parle à proximité, provoque très souvent une sélection non souhaitée. Ces dispositifs ne peuvent donc pas être utilisés de façon fiable en milieu ambiant bruyant.

La présente invention a notamment pour objectif de pallier ces inconvénients.

Un premier objectif de l'invention est de présenter un dispositif de sélection de canaux de commande multifonction permettant de sélectionner aisément, par le son, et notamment le souffle, une fonction parmi un grand nombre de fonctions sélectionnables.

Un autre objectif de l'invention est de permettre une telle sélection par le son, notamment le souffle, au moyen d'un dispositif et selon un processus qui permettent une grande sécurité de sélection.

Un objectif particulier de l'invention est de fournir un tel dispositif qui permette la génération de chaque chiffre d'un numéro de téléphone successivement, et la composition automatique d'un numéro préalablement mémorisé dans un "agenda" électronique sous un numéro de référence, par simple sélection "sonore" du numéro de référence.

Ces objectifs, ainsi que d'autres qui apparaîtront par la suite, sont atteints grâce à un dispositif de sélection de canaux de commande, notamment pour la commande d'appareils ou de fonctions telles que celles activables par le clavier d'un poste téléphonique, dispositif du type réagissant au son, et notamment au souffle, ce dispositif comprenant :
- des moyens (212) de commande d'au moins deux canaux (208) de commande distincts ;
- des moyens (203) de comptage d'impulsions (25), chaque valeur prise par lesdits moyens (203) de comptage correspondant à l'un desdits canaux (208), l'incrémentation desdits moyens (203) de comptage se faisant selon un ordre de présélection prédéterminé ;
- des moyens (22) de traitement d'un signal électrique (21) issu d'un capteur (20), produisant lesdites impulsions (25) lorsque ledit signal électrique (21) est représentatif d'un niveau de bruit minimum prédéterminé et/ou réglable ;
- des moyens (26) de temporisation permettant de générer un signal de sélection (201) lorsque les moyens (203) de comptage n'ont pas été réincrémentés depuis un laps de temps (T1) prédéterminé et/ou réglable ;
- des moyens (207) de transfert de la valeur (204) contenue dans lesdits moyens (203) de comptage vers lesdits moyens (212) de commande, lesdits moyens (207) de transfert étant actifs lorsque lesdits moyens (26) de temporisation génèrent ledit signal de sélection (201).

Ainsi ce n'est plus le son qui permet la sélection, mais l'absence de son. Il y a sélection uniquement lorsqu'aucun signal n'a été reçu pendant le temps T1. En d'autres termes, l'invention repose sur une approche nouvelle et inventive d'un dispositif de commande par le son, opposée à celles connues, consistant à :
- émettre un bruit continu ou quasi continu pour provoquer le défilement d'un compteur, dont chaque valeur correspond à un canal de commande ;
- effectuer la sélection d'un canal en interrompant le bruit de "progression" du compteur pendant une durée T1 suffisamment longue pour être significative d'une volonté de sélection de l'utilisateur (tout en lui laissant le temps de reprendre son souffle).

Cela apporte des conditions de sécurité et de validité qui n'existaient pas auparavant. Notamment, un bruit intempestif entraîne la reprise du comptage, mais aucune sélection non-voulue.

Par ailleurs, ce dispositif permet à l'utilisateur d'agir sur la présélection à son rythme : de brefs arrêts de souffle provoquent un ralentissement de l'incrémentation.

Avantageusement, ladite incrémentation des moyens de comptage est cyclique, la présélection du dernier desdits canaux selon ledit ordre de présélection prédéterminé étant suivi de la présélection du premier desdits canaux.

Préférentiellement, le capteur est un transducteur électro-acoustique sensible essentiellement au souffle, tel qu'un microphone à électret.

De façon avantageuse, le bloc de traitement du signal issu du capteur engendre des impulsions formées chacune à chaque détection d'un pic du signal issu du capteur d'une valeur supérieure à un seuil prédéterminé et/ou réglable.

Préférentiellement, dans le cas de l'utilisation d'un capteur de souffle, le bloc de traitement du signal électrique issu du capteur comprend des moyens de seuillage permettant de distinguer un souffle de commande d'autres sons.

Selon un mode de mise en oeuvre préférentiel de l'invention, le bloc de traitement du signal coopère avec des moyens de réinitialisation des moyens de comptage d'impulsions lorsqu'un nombre d'impulsions prédéterminé n'est pas atteint dans le laps de temps prédéterminé et/ou réglable.

Avantageusement, les moyens de comptage coopèrent avec des moyens de précomptage, lesdits moyens de précomptage étant sollicités en premier lors du début de chaque détection de son, et ne permettant l'activation desdits moyens de comptage que lorsque un nombre prédéterminé d'impulsions de précomptage a été enregistré pendant un temps prédéterminé.

Préférentiellement, le dispositif comporte des moyens de validation du canal sélectionné, activés par la sélection d'un canal spécifique de validation parmi les canaux présélectionnés.

Dans un mode de réalisation avantageux, le dispositif de l'invention permet la sélection de fonctions téléphoniques, du type des fonctions de composition d'un numéro d'appel téléphonique. Ce dispositif comprend alors des moyens de transcodage de la valeur prise par les moyens de comptage en un signal représentatif de la fonction téléphonique correspondante, selon une table de transcodage préétablie.

Selon autre caractéristique préférentielle, la table de transcodage associe à chaque valeur issue des moyens de comptage un mot alphanumérique complexe, notamment un numéro de téléphone, la table pilotant, pour chaque fonction sélectionnée, une unité délivrant aux moyens de comptage un jeu d'impulsions calibrées correspondant à cette fonction.

Avantageusement, le dispositif comprend des moyens de visualisation des opérations effectuées, sous la forme d'une paire d'afficheurs visuels pour chaque fonction sélectionnable, le premier afficheur de chaque paire indiquant que la fonction courante est présélectionnée et le second afficheur confirmant la sélection de la fonction correspondante.

D'autres caractéristiques et avantages de l'invention apparaîtront à la lecture de la description suivante d'un mode de réalisation préférentiel de l'invention, donné à titre illustratif et non limitatif, et des dessins annexés dans lequels :
- la figure 1 représente le signal issu d'un microphone à électret généré par un souffle en comparaison avec celui généré par de la parole ;
- la figure 2 représente un mode de mise en oeuvre préférentiel du dispositif selon l'invention appliqué à la génération d'un numéro de téléphone ;
- la figure 3A représente un jeu de monostables permettant une brève interruption du souffle de commande sans réinitialisation du dispositif ;
- la figure 3B représente l'état des sorties des monostables de la figure 3A et les commandes effectuées dans le cas d'un souffle continu puis haché ;
- la figure 4 est un exemple de représentation d'un cadran téléphonique adapté à la présente invention.

Le mode de réalisation de l'invention présenté ci-après est relatif à un dispositif de commande multifonctions sensible sélectivement au souffle. Un tel dispositif présente ainsi les avantages d'être utilisable en milieu bruyant, par tout opérateur (notamment handicapé) simplement capable d'expirer de l'air par la bouche pendant une durée contrôlable et variable.

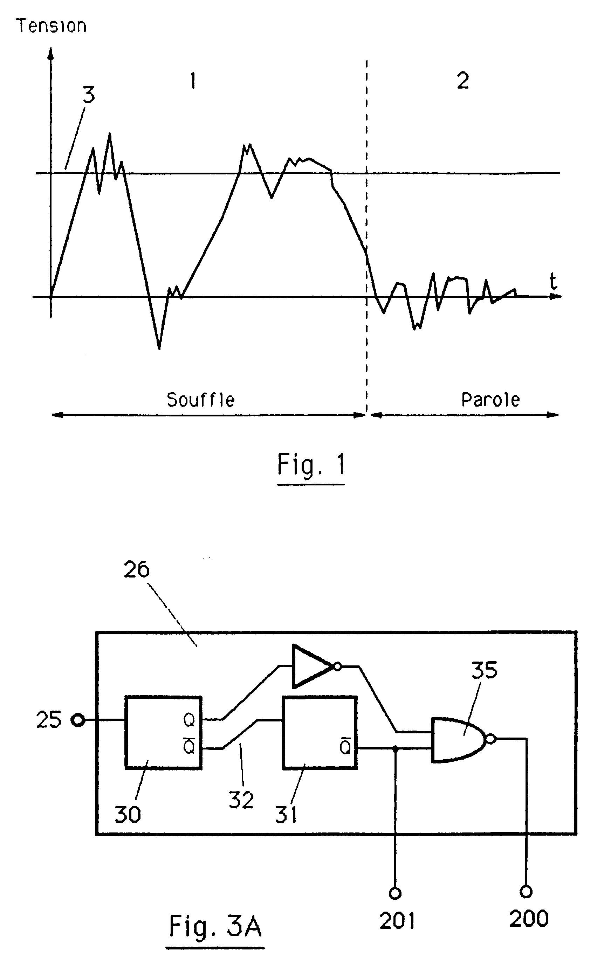

La figure 1 représente le signal détecté par un microphone à électret en présence d'un souffle (1), puis en présence de la parole (2).

Un microphone à électret délivre donc des signaux de niveaux très élevés dans le cas du souffle 1. Dans le cas d'une parole, de mots ou de sons 2 prononcés dans un tel microphone, l'amplitude délivrée est en revanche nettement inférieure à celle provenant d'un souffle 1.

Il apparaît donc qu'il est relativement aisé de distinguer le souffle 1 de la parole 2, par simple seuillage 3. Il est ainsi possible de fonctionner en milieu ambiant bruyant, le dispositif ne tenant compte que du souffle 2.

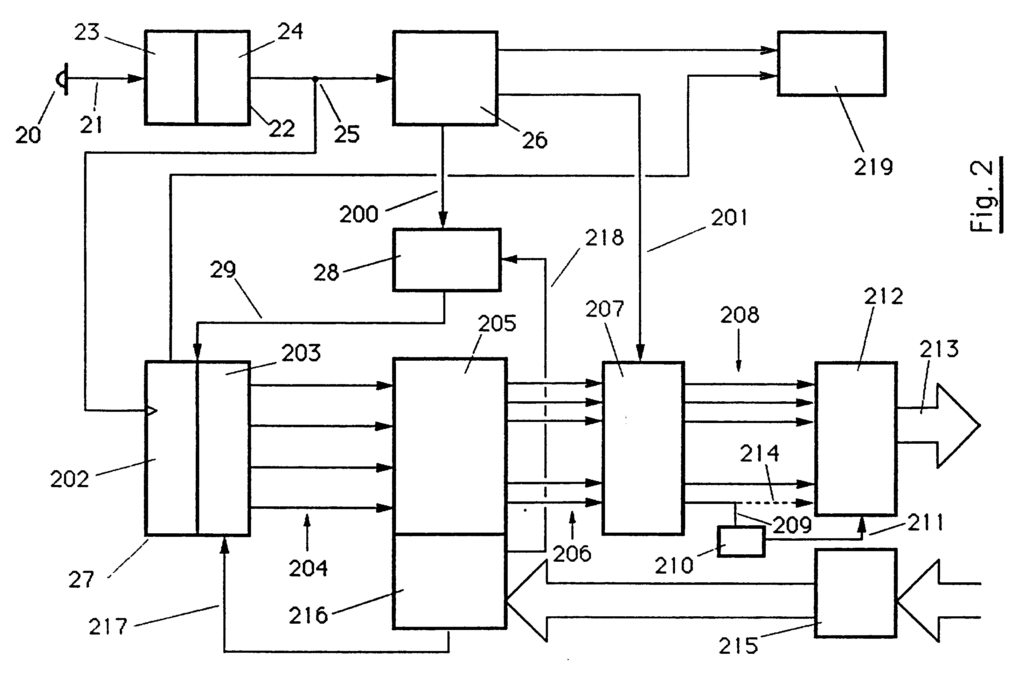

La figure 2 représente un mode de mise en oeuvre préférentiel du dispositif de l'invention. Ce mode de mise en oeuvre correspond à la génération d'un numéro de téléphone.

Le signal de souffle est capté par un microphone à électret 20 qui transforme le souffle perçu en un signal électrique dont l'allure est représentée en figure 1. Ce signal électrique est transmis 21 à un bloc 22 de traitement du signal comportant deux modules 23,24. Le premier module 23 effectue un filtrage et une mise en forme du signal issu du microphone à électret 20. Le second module 24 effectue une conversion en une horloge numérique du signal filtré et mis en forme. Cette conversion est réalisée par comparaison de l'amplitude du signal traité avec une valeur de seuil 3 pour générer une impulsion d'horloge à chaque franchissement de seuil.

Ce seuil 3 est représenté à la figure 1. Si l'amplitude du signal d'entrée 21 est supérieure à la valeur du seuil 3, un niveau logique "1" apparaît en sortie du module 24. Ce seuil 3 est ajusté à une valeur permettant de ne pas prendre en compte un signal de parole 2 (fig.1). Il peut également être réglable par l'utilisateur. Le signal de sortie 25 du bloc 22 de traitement correspond donc à une horloge logique dont la fréquence est fonction de chaque dépassement du niveau 3 du signal de souffle capté par le microphone à électret 20, et/ou de la durée de ces dépassements.

Dans le cas où l'on ne prend en compte que les dépassements de seuil, celui-ci est avantageusement choisi suffisamment élevé pour que, même en présence d'un souffle continu, il soit régulièrement franchi ainsi que cela est illustré en figure 1.

Ce signal d'horloge 25 est transmis simultanément à deux blocs : un bloc de temporisation 26 et un bloc de comptage 27.

Le bloc de temporisation 26 pilote notamment la réinitialisation du bloc de comptage 27, lorsqu'aucun souffle n'a été enregistré pendant une durée prédéterminée.

La figure 3A présente un exemple de bloc de temporisation constitué d'un jeu de monostables 30,31.

Dès l'apparition d'un front montant à l'entrée 25 du premier monostable 30, sa sortie inversée 32 passe à l'état bas. Cet état est maintenu pendant une certaine durée, qui est réglée, selon un premier mode de mise en oeuvre de la présente invention, de telle sorte que de brefs niveaux bas à l'entrée 25 de ce monostable 30 provoquent le passage de la sortie 32 à l'état haut.

Lorsqu'il n'y a plus d'impulsions à l'entrée 25 du premier monostable 30, la sortie inversée 32 passe donc au niveau haut. Le front montant provoque l'activation du second monostable 31 pendant une période T1 d'environ 2,2 secondes. Ce temps T1 ne constitue bien sûr qu'une valeur préférentielle adaptée à l'utilisation qui est faite du dispositif selon l'invention, et une autre valeur du même ordre de grandeur peut également convenir. Cette valeur peut notamment être ajustée par l'utilisateur.

L'apparition d'un nouveau front montant à l'entrée 25 du premier monostable 30, provoquée par une reprise du souffle dans le microphone à électret 20, pendant cette période T1, prolonge l'activation du monostable 31. La sortie 201 du deuxième monostable 31 reste ainsi active le temps de la présence du souffle augmenté du temps T1.

Après la fin de détection de souffle augmenté du temps T1, le signal issu du second monostable 31 est validé (il passe à 1) et provoque un ordre de sélection sur la liaison 201. Une porte NON-ET 35 commande la réinitialisation 29 du bloc de comptage des impulsions via les moyens de réinitialisation 28 (fig. 2), avec un retard permettant d'effectuer une sélection avant la réinitialisation 29.

Ce mode de réalisation consiste donc à utiliser la non-continuité du signal 25 (horloge), chaque nouveau dépassement du seuil 3 provoquant une prolongation de la validation du monostable 31 d'un temps T1.

Ce bloc de temporisation permet qu'une très brève interruption du souffle n'entraîne ni la réinitialisation complète du comptage ni une sélection non voulue. Il est ainsi possible de piloter le dispositif de l'invention par un souffle continu et/ou par une série de souffles. Cela permet notamment à l'utilisateur de "reprendre son souffle".

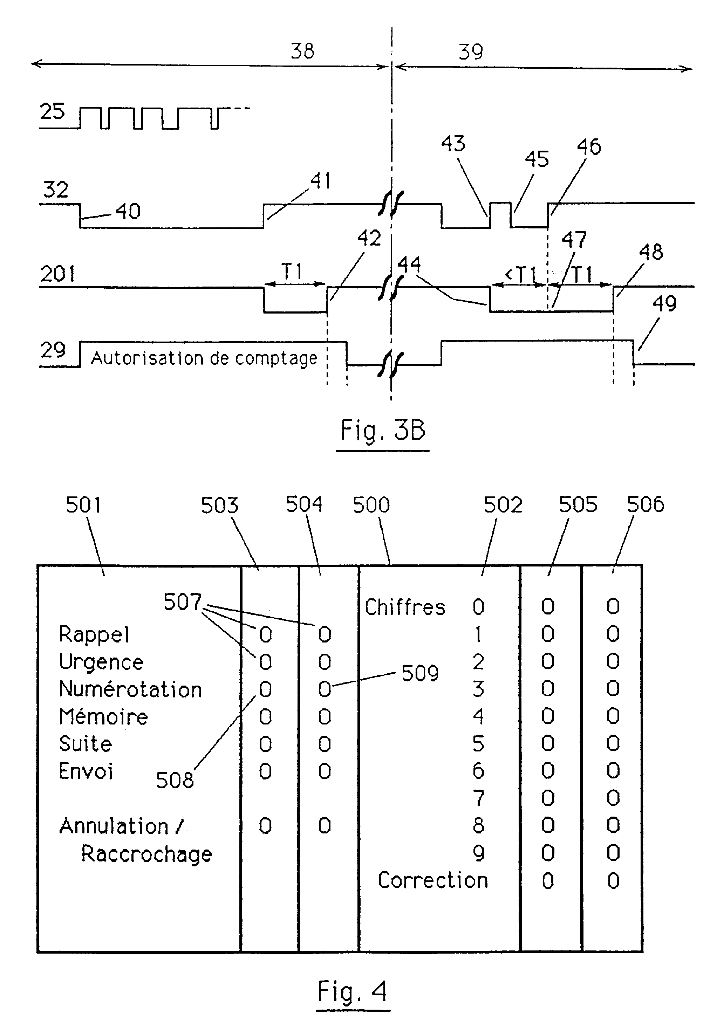

Le fonctionnement du dispositif est illustré en figure 3B, qui représente l'état des sorties des monostables 30,31, et les commandes effectuées 29,201 dans le cas d'un souffle continu 38 puis dans le cas d'un souffle avec reprises 39.

Le premier monostable 30 est déclenché 40 par l'apparition d'impulsions générées par un souffle.

A chaque interruption du signal 25 pendant un temps supérieur au temps de garde du monostable 30, le second monostable 31 est déclenché pendant un temps de garde T1 ou sa validation est prolongée d'une durée T1. A la fin de cette période T1, la sortie 201 du second monostable 31 passe à "1". Le front montant 42 généré valide la présélection effectuée (fig.2) et réinitialise 29 le bloc de comptage 27 d'impulsions à l'aide des moyens d'initialisation 28.

Dans le cas de reprises de souffle 39, chaque nouvelle interruption de souffle 43 génère l'activation 44 du second monostable 31. Si pendant la période T1 de nouvelles impulsions sont détectées à l'entrée du premier monostable 30, les ordres de sélection 201 et de réinitialisation 200 ne sont pas générés. Ceux-ci ne le seront que dans le cas d'une interruption de souffle de durée supérieure au temps de garde T1.

La reprise du souffle pendant cette période T1 génère en effet de nouvelles impulsions à l'entrée 25 du bloc de temporisation et redéclenche 45 le premier monostable 30.

En revanche, pendant ce temps T1 le premier monostable 30 n'est plus activé, le second monostable 31 se désactive au bout du temps T1 et provoque un ordre de sélection 48 sur la liaison 201 puis, à l'aide du bloc d'initialisation 28, un ordre d'initialisation 49.

Ce mode de mise en oeuvre permet donc d'interrompre le souffle pendant un temps inférieur ou égal à T1 sans effectuer de sélection. Bien entendu, il suppose que le signal d'horloge 25 soit d'une fréquence supérieure à une certaine valeur de telle sorte qu'une interruption de souffle d'environ seconde soit permise (pour un temps de garde T1 de 2,2s), afin de permettre une prolongation d'un nouveau temps de garde T1.

Selon un autre mode de réalisation (non représenté), la durée de maintien du temps bas par ce monostable 30 est choisie de telle sorte que de brefs niveaux bas à l'entrée 25 ne provoquent pas le passage de la sortie 32 à l'état haut.

Ainsi, lors d'une interruption de souffle, l'utilisateur dispose, après l'interruption de souffle, de l'intégralité du temps de garde T1 pour recommencer à souffler dans le capteur 20.

Ce mode de mise en oeuvre présente l'avantage d'offrir une meilleure fiabilité du dispositif selon l'invention, notamment dans le cas d'un souffle de puissance élevée (temps haut du signal 25 importants) et de durée proche du temps de garde T1.

En effet, une configuration spécifique (non représentée) du bloc de temporisation 26 permet de prendre en compte les signaux de souffle 25 possédant un temps haut (d'activation) de durée proche de T1, sans effectuer de sélection. Elle offre de plus la possibilité d'interrompre un souffle pendant un temps très proche de T1, puisqu'on ne compte pas sur une certaine fréquence du signal 32 pour le déclenchement du monostable 31. Ainsi, c'est uniquement le premier front montant du signal 25 qui est pris en compte pour prolonger la durée de maintien du monostable 31 réalisant l'opération de sélection.

La figure 2 ainsi que la description qui va suivre permettront de mieux comprendre l'utilité de ce dispositif de temporisation.

Les impulsions 25 générées par le bloc de traitement 22 sont transmises, outre au bloc de temporisation 26, aux moyens 27 de comptage d'impulsions. Ces moyens seront constitués d'un module de précomptage 202 et d'un module de comptage 203.

Le module de précomptage 202 permet d'ajuster la sensibilité du système aux difficultés possibles à souffler de l'usager, pour rendre la commande plus ou moins sensible, au détriment de la sélectivité du souffle. Le module de précomptage 202 constitue également une précondition de comptage, permettant de minimiser les risques de déclenchement intempestifs causés par des bruits brefs, par exemple des claquements de porte.

Les moyens de précomptage 202 ne fournissent des impulsions au compteur 203 qu'une fois qu'un certain nombre d'impulsions auront été précomptées dans l'intervalle de temps T1. Le module de précomptage 202 constitue ainsi un seuil de comptage minimal à dépasser pour que les impulsions soient prises en compte par le compteur 203.

Les moyens 203 de comptage d'impulsions 25 sont par ailleurs réinitialisés, ainsi qu'on l'a vu, lorsqu'un nombre d'impulsions prédéterminé n'est pas atteint dans le laps de temps (T1) prédéterminé et/ou réglable.

Le bloc de comptage 27 est donc initialisé par le bloc de temporisation 26 via des moyens de réinitialisation 28. Dans le bloc 27, le module 203 compte le nombre d'impulsions qui lui sont fournies par le module de précomptage 202. Il présente sur ses sorties 204 un mot binaire représentatif du nombre d'impulsions comptées. Ce mot binaire est décodé par un module de décodage 205, qui associe à ce mot binaire la fonction ou le chiffre présélectionné correspondant.

Il ne s'agit bien sûr que d'une présélection, qui n'est validée que lorsqu'un laps de temps T1 sans souffle s'est écoulé. Le module de décodage 205 suit donc en permanence l'évolution du compteur.

Dans un autre mode de réalisation, on peut prévoir que le module de décodage 205 ne réalise le décodage que lorsque le signal de validation 201 est émis.

Le module 205 possède à sa sortie un certain nombre de canaux 206 dont un seul est activé (présélectionné) à la fois.

Ce canal est celui du chiffre ou de la fonction courante présélectionné à l'aide du capteur de souffle 20. Comme décrit précédemment, lorsque le canal courant n'a pas été modifié après une interruption de souffle de durée supérieure au temps de garde T1 du second monostable 31, le canal présélectionné est sélectionné grâce au bloc de transfert 207 qui recopie sur ses sorties 208 les niveaux présents sur ses entrées 206, lorsqu'il détecte un ordre de transfert 201 généré par le bloc de temporisation 26.

Avant de pouvoir réaliser les commandes proprement dites, il peut être intéressant de valider le canal sélectionné. Cette validation est effectuée par la sélection d'un canal particulier, appelé canal de validation 209. La sélection de ce canal et le traitement de l'information par un module de gestion 210 assure ensuite la validation 211 de la sélection déjà transmise au bloc de commande 212. Ce bloc de commande 212 envoie alors sur les canaux de commande 213 les ordres correespondants à l'appareil à piloter.

Dans le cas de la génération d'un numéro de téléphone par exemple, il serait fastidieux d'avoir à valider chaque chiffre sélectionné. C'est pourquoi le bloc de commande 212 peut être avantageusement constitué par une unité à microprocesseur gérant les canaux sélectionnés 208. Ceci permet de sélectionner tous les chiffres du numéro de téléphone successivement et d'effectuer par la suite une seule opération de validation 211.

L'utilisation d'un microprocesseur pour la gestion des canaux sélectionnés 208, permet de plus de se passer du module de gestion 210 du canal de validation 209 et de valider une commande directement par le canal 214.

Dans de nombreux dispositifs, et notamment dans le cas d'un appareil téléphonique, il peut être intéressant de distinguer plusieurs zones, ainsi qu'on le verra par la suite, en relation avec la figure 4. Il est possible alors de choisir l'une des zones de progression, pour la sélection.

Ainsi, par exemple, la génération d'un numéro de téléphone par sélection successive des chiffres composant ce numéro est réalisée dans la zone de progression principale. Les autres zones correspondent à la sélection de fonctions particulières. Le fait de gérer différentes zones de progression permet de simplifier le dialogue avec l'utilisateur, et de limiter le temps de sélection (il n'est pas nécessaire de balayer l'ensemble des canaux susceptibles d'être sélectionnés).

Le bloc 215 permet de sélectionner des zones de comptage et des mots de validation. Ce bloc 215 peut être programmé soit par logiciel, soit par des circuits logiques. Il permet de boucler sur un certain nombre de fonctions permettant d'accéder à des zones différentes suivant les applications du dispositif. La programmation de ce bloc permet également de boucler dans chaque zone sélectionnée.

Les données provenant de ce bloc 215 sont transcodées par un module 216. Le programme permet de générer un certain nombre d'impulsions sur une ligne 217 raccordée au bloc de comptage 203 précédemment réinitialisé, par l'intermédiaire du module 28 d'initialisation, à partir d'une commande 218 provenant du module de transcodage 216. Le nombre d'impulsions générées apparaît à la sortie du module de comptage sur les lignes 204 raccordées au bloc de décodage 205 et le processus précédemment décrit est répété dans une autre zone de progression.

La figure 4 est un exemple de représentation d'un cadran téléphonique adapté à la présente invention.

Ce cadran 500 est partagé en deux parties, correspondant globalement aux fonctions 501 et aux chiffres 502. Il présente deux rampes de deux paires d'afficheurs 503,504,505,506. Ces rampes constituent une interface utilisateur-dispositif permettant à l'utilisateur de visualiser les opérations effectuées.

Les rampes 503,505 et 504,506 sont constituées par exemple de diodes électroluminescentes 507 connectées respectivement aux canaux de présélection 206 et de sélection 208.

Si l'utilisateur veut composer un numéro chiffre à chiffre, il actionne le capteur 20 (en l'occurence le capteur de souffle), ce qui a pour effet d'allumer tour à tour et pendant quelques fractions de secondes les diodes électroluminescentes 507 de la rampe 503. Lorsqu'il atteint la diode électroluminescente 508 correspondant à NUMEROTATION, il s'arrête un instant. La diode électroluminescente 509 située en face de celle précédemment illuminée 508 s'allume à son tour dans la rampe 504 au bout du temps de garde T1 du second monostable 31 de la figure 3A. La fonction NUMEROTATION est alors sélectionnée, sans toutefois être validée, ce qui permet de pouvoir sélectionner une autre fonction par une nouvelle sollicitation du capteur 20, si le choix de la sélection précédente était une erreur.

Afin de valider la fonction sélectionnée, il faut sélectionner la fonction ENVOI de la même façon que précédement. La procédure est poursuivie par une nouvelle sollicitation du capteur 20 pour présélectionner un chiffre sur la rampe 505. Au bout du temps de garde T1, le chiffre présélectionné est sélectionné et la diode électroluminescente située en face de celle indiquant le chiffre présélectionné s'illumine. Cette procédure est à exécuter autant de fois qu'il y a de chiffres dans le numéro de téléphone à composer. Des moyens de visualisation du numéro en cours de composition sont avantageusement disposés à proximité des rampes. Une fois le dernier chiffre du numéro sélectionné, le numéro complet est validé par la fonction ENVOI. Ce numéro est alors généré par l'unité à microprocesseur compris dans le bloc de commande.

Pour appeler un numéro en mémoire, il suffit de sélectionner la fonction MEMOIRE et son numéro d'ordre dans la mémoire.

Pour un appel d'urgence, il suffit de sélectionner la fonction URGENCE et de la valider par ENVOI.

Le dispositif selon l'invention peut également comporter un bloc de visualisation 219 (fig. 2) informant l'utilisateur que son signal de souffle est bien pris en compte.

L'appel de toutes les fonctions n'est validé qu'après ENVOI. Vu le positionnement de cette fonction (valeur maximale du compteur) le système est difficilement déclenchable par une perturbation quelconque. Sa sélection est réalisée par simple "amenée en butée" du dispositif de comptage au moyen d'un souffle de commande suffisamment long. Bien entendu, ceci suppose que le dispositif de comptage vienne en butée à sa valeur maximale, ce qui ne constitue qu'un mode de réalisation possible de l'invention.

Dans un autre mode de réalisation d'un dispositif de comptage, le comptage est cyclique déroulant, c'est à dire qu'une incrémentation de la valeur maximale du compteur fait repasser ce compteur à zéro.

Les différentes fonctions réalisables par le dispositif selon l'invention, ainsi que le nombre et les fonctions des zones de progression, ne sont pas limitatives. Par exemple, la figure commentée comporte notamment les fonctions RAPPEL permettant de rappeler le dernier numéro généré, URGENCE permettant de générer des numéros propres aux services du type police, pompiers, etc..., par sélection d'un numéro d'ordre dans une liste et CORRECTION permettant d'effacer un chiffre sélectionné.

Bien entendu, le dispositif selon l'invention peut être appliqué à la commande d'autres appareils que des postes téléphoniques.

Le type de transducteur utilisé ne constitue pas un choix critique, tout transducteur générant un signal tout le temps de sa mise en fonction peut être utilisé. Suivant le type de capteur utilisé, il conviendra peut-être de modifier la structure du bloc de traitement 22, afin de l'adapter aux types de signaux électriques fournis par le capteur.

Les moyens de visualisation peuvent être également constitues d'indicateurs mécaniques ou de systèmes utilisant des synthétiseurs de parole.

Par ailleurs, les figures 1, 2, 3A, 3B et 4 ne représentent bien sûr qu'un mode de mise en oeuvre spécifique de l'invention. L'homme de métier imaginera aisément d'autres applications entrant dans le cadre de la présente invention.

## Revendications

1. Dispositif de sélection de canaux de commande, notamment pour la commande d'appareils ou de fonctions telles que celles activables par le clavier d'un poste téléphonique, dispositif du type réagissant au son, et notamment au souffle,
caractérisé en ce qu'il comprend :
- des moyens (212) de commande d'au moins deux canaux (208) de commande distincts ;
- des moyens (203) de comptage d'impulsions (25), chaque valeur prise par lesdits moyens (203) de comptage correspondant à l'un desdits canaux (208), l'incrémentation desdits moyens (203) de comptage se faisant selon un ordre de présélection prédéterminé ;
- des moyens (22) de traitement d'un signal électrique (21) issu d'un capteur (20), produisant lesdites impulsions (25) lorsque ledit signal électrique (21) est représentatif d'un niveau de bruit minimum prédéterminé et/ou réglable ;
- des moyens (26) de temporisation permettant de générer un signal de sélection (201) lorsque les moyens (203) de comptage n'ont pas été réincrémentés depuis un laps de temps (T1) prédéterminé et/ou réglable ;
- des moyens (207) de transfert de la valeur (204) contenue dans lesdits moyens (203) de comptage vers lesdits moyens (212) de commande, lesdits moyens (207) de transfert étant actifs lorsque lesdits moyens (26) de temporisation génèrent ledit signal de sélection (201).

2. Dispositif selon la revendication 1, caractérisé en ce que ladite incrémentation des moyens (203) de comptage est cyclique, la présélection du dernier desdits canaux selon ledit ordre de présélection prédéterminé étant suivi de la présélection du premier desdits canaux.

3. Dispositif selon l'une quelconque des revendications 1 ou 2, caractérisé en ce que ledit capteur (20) est un transducteur électro-acoustique sensible essentiellement au souffle, tel qu'un microphone à électret.

4. Dispositif selon l'une quelconque des revendications 1 à 3, caractérisé en ce que lesdits moyens (22) de traitement du signal (21) issu du capteur (20) engendrent des impulsions (25) formées chacune à chaque détection d'un pic du signal issu du capteur (20) d'une valeur supérieure à un seuil (3) prédéterminé et/ou réglable.

5. Dispositif selon l'une quelconque des revendications 1 à 4, caractérisé en ce que lesdits moyens (22) de traitement du signal coopèrent avec des moyens (28) de réinitialisation desdits moyens (203) de comptage d'impulsions (25) lorsqu'un nombre d'impulsions prédéterminé n'est pas atteint dans ledit laps de temps prédéterminé (T1) et/ou réglable.

6. Dispositif selon l'une quelconque des revendications 1 à 5, caractérisé en ce que lesdits moyens (203) de comptage coopèrent avec des moyens (202) de précomptage, lesdits moyens (202) de précomptage étant sollicités en premier lors du début de chaque détection de son, et ne permettant l'activation desdits moyens (203) de comptage que lorsque un nombre prédéterminé d'impulsions de précomptage a été enregistré pendant un temps prédéterminé.

7. Dispositif selon l'une quelconque des revendications 1 à 6, caractérisé en ce qu'il comporte des moyens (212) de validation du canal (208) sélectionné, activés par la sélection (201) d'un canal spécifique de validation parmi lesdits canaux (206).

8. Dispositif selon l'une quelconque des revendications 1 à 7, caractérisé en ce qu'il est du type permettant la sélection de fonctions téléphoniques, et notamment de composition d'un numéro d'appel téléphonique, et en ce qu'il comprend des moyens (216) de transcodage de la valeur prise par lesdits moyens (203) de comptage en un signal représentatif de la fonction téléphonique correspondante, selon une table de transcodage préétablie.

9. Dispositif selon la revendication 8, caractérisé en ce que ladite table de transcodage associe à chaque valeur des moyens (203) de comptage un mot alphanumérique complexe, notamment un numéro de téléphone,
et en ce que ladite table pilote, pour chaque fonction sélectionnée, une unité délivrant auxdits moyens (203) de comptage un jeu d'impulsions calibrées (217) correspondant à ladite fonction.

10. Dispositif selon l'une quelconque des revendications 1 à 9, caractérisé en ce qu'il comprend des moyens (503,504,505,506) de visualisation des opérations effectuées, sous la forme d'une paire (508,509) d'afficheurs visuels par fonction sélectionnée, le premier afficheur (508) de chaque paire indiquant la fonction courante présélectionnée et le second afficheur confirmant la sélection de la fonction correspondante.

## Patentansprüche

1. Vorrichtung zur Auswahl von Steuerungskanälen, insbesondere zum Steuern von Geräten oder von Funktionen der Art, die über die Tastatur eines Telefons aktivierbar sind, wobei es sich um eine Vorrichtung handelt, die auf Schall und insbesondere auf Atemgeräusche reagiert, dadurch gekennzeichnet, daß sie folgendes umfaßt:
- Mittel (212) zum Steuern von mindestens zwei verschiedenen Steuerkanälen (208);
- Mittel (203) zum Zählen von Impulsen (25), wobei jeder Wert, den die Zählmittel (203) annehmen einem der Kanäle (208) entspricht, und wobei die Inkrementierung der Zählmittel (203) nach einer vorher festgelegten Auswahlordnung erfolgt;
- Mittel (22) zur Bearbeitung eines elektrischen Signals (21), das aus einem Meßfühler (20) kommt und die Impulse (25) produziert, wenn das elektrische Signal (21) repräsentativ für einen vorgegebenen und/oder regelbaren minimalen Geräuschpegel ist;
- Verzögerungsmittel (26), die das Erzeugen eines Auwahlsignals (201) ermöglichen, wenn die Zählmittel (203) nach einem vorher festgelegten und/oder regelbaren Zeitintervall (T1) nicht wieder inkrementiert wurden;
- Mittel (207) für die Übertragung des in den Zählmitteln (203) enthaltenen Wertes (204) zu den Steuerungsmitteln (212), wobei die Übertragungsmittel (207) aktiv sind, wenn die Verzögerungsmittel (26) das Auswahlsignal (201) erzeugen.

2. Vorrichtung gemäß Anspruch 1,
dadurch gekennzeichnet, daß die Inkrementierung der Zählmittel (203) zyklisch ist, wobei auf die Vorauswahl des letzten Kanals gemäß der Vorauswahlordnung, die Vorauswahl des ersten Kanals folgt.

3. Vorrichtung gemäß einem der Ansprüche 1 oder 2,
dadurch gekennzeichnet, daß es sich beim Meßfühler (20) um einen elektroakustischen Wandler handelt, der vornehmlich auf Atemgeräusche reagiert, wie beispielsweise ein Elektretmikrofon.

4. Vorrichtung gemäß einem der Ansprüche 1 bis 3,
dadurch gekennzeichnet, daß die Mittel (22) zur Bearbeitung des Signals (21), das aus dem Meßfühler (20) kommt, Impulse (25) erzeugen, die jeweils bei der Erfassung einer Spitze des aus dem Meßfühler (20) kommenden Signals gebildet werden, und deren Wert oberhalb eines im voraus festgelegten und/oder regelbaren Schwellwerts (3) liegt.

5. Vorrichtung gemäß einem der Ansprüche 1 bis 4,
dadurch gekennzeichnet, daß die Mittel (22) zur Signalbearbeitung mit den Mitteln (28) zur Reinitialisierung der Mittel (203) zum Zählen der Impulse (25) zusammenarbeiten, wenn eine im voraus festgelegte Zahl von Impulsen nicht innerhalb des vorgegebenen und/oder regelbaren Zeitintervalls (T1) erreicht wurde.

6. Vorrichtung gemäß einem der Ansprüche 1 bis 5,
dadurch gekennzeichnet, daß die Zählmittel (203) mit Mitteln (202) zum Vorauszählen zusammenarbeiten, wobei die Mittel (202) zum Vorauszählen als erstes beim Einsetzen einer jeden Schallerfassung aufgerufen werden und die Aktivierung der Zählmittel (203) nur dann zulassen, wenn eine im voraus gegebene Zahl von Impulsen zum Vorauszählen während einer vorgegebenen Zeit registriert wurde.

7. Vorrichtung gemäß einem der Ansprüche 1 bis 6,
dadurch gekennzeichnet, daß sie über Mittel (212) zum Validieren des ausgewählten Kanals (208) verfügt, welche durch die Auswahl (201) eines spezifischen Validierungskanals unter den Kanälen (206) aktiviert werden.

8. Vorrichtung gemäß einem der Ansprüche 1 bis 7,
dadurch gekennzeichnet, daß sie von der Art ist, welche die Auswahl von Telefonfunktionen ermöglicht, insbesondere das Wählen einer Telefonnummer und,
daß sie über Mittel (216) zum Umkodieren des von den Zählmitteln (203) angenommenen Wertes zu einem Signal verfügt, das repräsentativ für die entsprechende Telefonfunktion ist, gemäß einer im voraus festgelegten Umkodierungstabelle.

9. Vorrichtung gemäß Anspruch 8,
dadurch gekennzeichnet, daß die Umkodierungstabelle jedem Wert der Zählmittel (203) ein komplexes alphanumerisches Wort zuordnet, insbesondere eine Telefonnummer und,
daß die Tabelle für jede gewählte Funktion eine Einheit steuert, die den Zählmitteln (203) einen Satz kalibrierter Impulse (217) zur Verfügung stellt, welche dieser Funktion entsprechen.

10. Vorrichtung gemäß einem der Ansprüche 1 bis 9,
dadurch gekennzeichnet, daß sie über Mittel (503, 504, 505, 506) verfügt, um die ausgeführten Operationen sichtbar zu machen, in der Form eines Paars (508, 509) von Anzeigeeinheiten für jede ausgewählte Funktion, wobei die erste Anzeigeeinheit (508) eines jeden Paars die laufende vorgewählte Funktion angibt, und die zweite Anzeigeeinheit die Wahl der entsprechenden Funktion bestätigt.

## Claims

1. Device for selecting control channels, especially for the control of apparatuses or of functions such as those which can be activated by the keypad of a telephone set, a device of the type reacting to sound, and especially to breathing,
characterized in that it comprises:
- means (212) of control of at least two distinct control channels (208);
- means (203) of counting pulses (25), each value taken by the said means (203) of counting corresponding to one of the said channels (208), the incrementation of the said means (203) of counting being carried out according to a predetermined preselection order;
- means (22) for processing an electrical signal (21) from a sensor (20), producing said pulses (25) when the said electrical signal (21) is representative of a predetermined and/or adjustable minimum noise level;
- timer means (26) making it possible to generate a selection signal (201) when the means (203) of counting have not been reincremented for a predetermined and/or adjustable time span (T1);
- means (207) of transfer of the value (204) contained in the said means (203) of counting to the said means (212) of control, said means (207) of transfer being active when the said timer means (26) generate the said selection signal (201).

2. Device according to Claim 1, characterized in that the said incrementation of the means (203) of counting is cyclic, the preselection of the last of the said channels according to the said predetermined preselection order being followed by the preselection of the first of the said channels.

3. Device according to either one of Claims 1 or 2, characterized in that the said sensor (20) is an electro-acoustic transducer sensitive essentially to breathing, such as an electret microphone.

4. Device according to any one of Claims 1 to 3, characterized in that the said means (22) for processing the signal (21) from the sensor (20) give rise to pulses (25) each formed with each detection of a peak in the signal from the sensor (20) of a value greater than a predetermined and/or adjustable threshold (3).

5. Device according to any one of Claims 1 to 4, characterized in that the said means (22) for processing the signal cooperate with means (28) of reinitialization of the said means (203) of counting pulses (25) when a predetermined number of pulses has not been reached in the said predetermined and/or adjustable time span (T1).

6. Device according to any one of Claims 1 to 5, characterized in that the said means (203) of counting cooperate with means (202) of precounting, the said means (202) of precounting being first summoned at the start of each detection of sound, and allowing activation of the said means (203) of counting only when a predetermined number of precounting pulses has been recorded over a predetermined time.

7. Device according to any one of Claims 1 to 6, characterized in that it includes means (212) of enabling the selected channel (208), and which are activated by the selection (201) of a specific enabling channel from among the said channels (206).

8. Device according to any one of Claims 1 to 7, characterized in that it is of the type allowing the selection of telephone functions, and especially the dialing of a telephone call number, and in that it comprises means (216) for transcoding the value taken by the said means (203) of counting into a signal representative of the corresponding telephone function, according to a prespecified transcoding table.

9. Device according to Claim 8, characterized in that the said transcoding table associates a complex alphanumeric word, especially a telephone number, with each value of the means (203) of counting, and in that the said table governs, for each selected function, a unit delivering to the said means (203) of counting a string of calibrated pulses (217) corresponding to the said function.

10. Device according to any one of Claims 1 to 9, characterized in that it comprises means (503,504,505, 506) of visualizing the operations performed, in the form of a pair (508,509) of visual displays per selected function, the first display (508) of each pair indicating the preselected current function and the second display confirming the selection of the corresponding function.
